# EUROPEAN PATENT APPLICATION

(11) **EP 2 258 368 A2**
(43) Date of publication of application: **08.12.2010**
(21) Application number: 10180035.7
(22) Date of filing: 01.03.2004
(51) Int. Cl.: A61K 31/48, A61K 31/485, A61K 9/19

(54) **Pharmaceutical compositions for nasal delivery**

(30) Priority: 28.02.2003 GB 0304636
(62) Divisional of application: 04715947.0
(71) Applicant: BRITANNIA PHARMACEUTICALS LIMITED, Redhill, Surrey RH1 6YS (GB)
(72) Inventor: Merkus, Franciscus, B-2460, Kasterlee (BE); Lambert, Peter, Newbury, Berkshire RG14 1JN (GB); Adams, Gerald, Newbury, Berkshire RG14 1JN (GB)
(74) Representative: Haile, Alison Victoria

(57) **Abstract**

According to the invention there is provided a powdered pharmaceutical formulation suitable for nasal delivery which is a freeze-dried blend of active material and excipient(s) containing: 0.5 - 50% by wt of active material 50 - 99.5% by wt of excipient(s), and in which at least 0.1 % by wt of the blend is an amorphous state, which can be directly obtained by freeze drying without the need for milling and without containing the pronounced low particles defined as finings. Such powders retain free-flowing properties on storage, are physically and chemically stable and are readily soluble.

## Description

The present invention relates generally to formulations suitable for nasal delivery of pharmacologically and therapeutically active agents for systemic activity, in particular for nasal powders containing drugs such as apomorphine and dihydroergotamine and their salts.

It is widely recognized that administering drugs by the nasal route is advantageous in that the drug can be absorbed rapidly into the blood supply through the nasal tissue. The drug can then travel systemically to target sites remote from the nasal cavity and provide a therapeutic effect at the target site. Nasal delivery may also be used for active agents which act by inducing a localized therapeutic response within the nasal tissue e.g. an immunological response to reduce localized allergic reactions such as rhinitis.

For example, U.S. Patent No. 5,756,483 issued to Merkus, which is hereby incorporated by reference, discloses the intranasal delivery of a variety of compositions, including apomorphine and dihydroergotamine, in powder form, for example in combination with a cyclodextrin and/or a disaccharide and/or a polysaccharide and/or a sugar alcohol, for treating several diseases and/or disease symptoms.

For nasal delivery of a drug, the drug is usually dispersed in an aqueous vehicle to facilitate delivery. Most commonly a liquid vehicle is used in which the active agent is dissolved and delivered to the nasal cavity as an aqueous nasal spray or via a metered propellant system. While liquid formulations are simply delivered, they have a number of limitations. The dose of poorly water soluble drugs that can be delivered is limited, compounds may have limited stability in solution (requiring the inclusion of stabilizing agents) and generally preservatives are required to maintain microbiological quality. Preservatives may cause toxic or allergic reactions and/or disturb the mucociliary clearance of the nose. The use of powder formulations can provide advantages in these areas as the deliverable dose can be less limited, powders are inherently stable and there is no requirement for preservatives.

A powder formulation of an active agent must have certain characteristics for effective administration via the nasal cavity. The powder formulation must be (1) free flowing to facilitate processing and efficient delivery from a delivery system, (2) of a suitable particle size in order that particles are comfortably retained in the nasal cavity without significant deposition further into the respiratory tract, and (3) rapidly soluble in the nasal mucosa to enable rapid and complete absorption.

A suitable mean particle size is in the range of from 5 to 150µm. This is because particles of a size less than 5 µm may be deposited partly in the lung (N.Mygind, Nasal Allergy, 2nd Edition 1981, Blackwell Scientific Publications, London, pages 45-47). On the other hand, particles having a size greater than about 150µm may induce the sneeze reflex causing ejection of the entire dose from the nasal cavity and are too large to be dissolved quickly in the aqueous mucosal layer in the nose resulting in removal by nasal mucociliary clearance .(Marttin et al. Nasal mucociliary clearance as a factor in nasal drug delivery, Adv. Drug Deliv. Reviews 19, 1998, 13-38).

It will be understood that a powder sample will usually have a range of particle sizes distributed around a mean particle size. Thus not only has the mean particle size to be in the range of from 5 to 150µm but also the amounts of particles with a size less than 5µm or greater than 150µm need to be minimised. In other words, the particle size distribution curve needs to be narrow such that the particle size distribution is not polydisperse.

It is necessary for a formulation to be rapidly soluble in the nasal mucosa because of the natural clearance processes which occur in the nose to remove unwanted material from it. It takes approximately 15 minutes for any deposited material to be removed into the back of the patient's mouth.

Previously the preparation of a powder formulation to meet these criteria could be time consuming and uncertain. US patent 5,756,483 mentions freeze-drying as a possible technique for solvent removal after mixing drugs and excipients in solution, and proposes milling to obtain suitable particle sizes.

Physically a crystalline matrix provides a more straightforward system for freeze drying since crystalline materials are simple to assay, present few problems during processing and can provide a very shelf-stable product. For respiratory purposes they provide a dried matrix where individual particles absorb only a finite amount of moisture when exposed to a damp atmosphere and the particle will remain unchanged during storage. In general therefore a crystalline matrix is preferred for freeze-drying. However, when a freeze-dried product is intended for nasal application, the particles should be such that the particle size is optimized for efficient nasal absorption. By variation of the freezing and drying conditions, particle size can be adjusted to a limited extent. However crystalline matrices often display a size distribution which exhibits a pronounced low particle size tail defined as finings, which cannot be prevented totally by altering freezing or drying conditions. These finings may induce undesirable effects such as lung deposition of very small particles.

In contrast an amorphous matrix when exposed to atmospheric conditions will continue to reabsorb moisture continuously, ultimately forming a sticky residue within the nasal applicator. Consequently amorphous matrices present difficulties when producing a drug for respiratory administration. The active component may be amorphous or may require stabilization of its molecular structure by including amorphous protectants in the formulation.

A further problem is that production of particles by milling tends to increase the amount of finings (particles having a size less than 10µm) which is unacceptable for the reasons given above.

The present invention is based on the surprising finding that by selection of components with a specific crystalline/amorphous balance, powder formulations of active materials and excipients of suitable particle size for nasal delivery can be directly obtained by freeze drying without the need for milling and without containing the pronounced low particles defined as finings. Such powders retain free-flowing properties on storage, are physically and chemically stable and are readily soluble.

In its broadest aspect, the present invention provides a powdered pharmaceutical formulation for nasal delivery which is a freeze-dried blend of active material(s) and excipient(s) containing:
0.5 - 50 % by wt of the active material(s),
50 - 99.5 % by wt of excipient(s),
   and in which at least 0.1 % by wt of the blend is an amorphous state.

Suitably from 0.1, preferably from 0.5, more preferably from 1 to 50 % by wt of the blend is in an amorphous state.

For example one formulation of the invention may contain:
0.5 - 50 % by wt of active material in amorphous state,
50 - 99.5 % by wt of excipient in crystalline state.
0 - 5 % by wt of excipient in amorphous state.

For example another formulation of the invention may contain:
0.5 - 50 % by wt of active material in crystalline state,
50 - 99.4 % by wt of excipient in crystalline state.
0.1 - 5 % by wt of excipient in amorphous state.

More specifically a formulation of the invention may contain:
0.5 - 25 % by wt of amorphous or crystalline state of active material,
75 - 99 % by wt of crystalline state excipient,
0.5 - 5 % by wt of amorphous state excipient.

A small (from 0.1 to 10% w/w, preferably from 0.1 to 1% w/w) amount of an additive/stabilizer for the active material, such as an antioxidant, may be included if desired, within the overall crystalline/amorphous balance.

The freeze dried blend of excipient(s) and active material(s) according to the invention is generally in composition identical to that of an aqueous solution of excipient(s) and active material(s). Therefore an average particle of a formulation according to the invention will contain active material and excipient in the same percentage amounts as their percentages in the original solution.

The crystalline/amorphous character of active material(s) and excipient(s) intended for freeze-drying in accordance with this invention may be assessed as three groups:
1. Active material(s) and excipient(s) which are crystalline and which persist in this form throughout freeze drying to provide a dried, crystalline matrix. The excipient(s) may be defined as crystalline and may be selected from a eutectic salt (such as sodium chloride, potassium chloride), certain amino acids (such as glycine), certain sugar alcohols (such as mannitol and sorbitol), and other organic molecules.
2. Active material(s) and excipient(s) which are crystalline but which may be induced into a non-crystalline or amorphous state by freezing and once in this state remain amorphous throughout subsequent drying, final finishing, storage and distribution. Examples include certain amino acids (such as glutamine or serine), a monosaccharide (such as glucose), a disaccharide (such as sucrose, trehalose, lactose), a trisaccharide (such as raffinose), a polysaccharide, certain polyethylene glycols (such as polyethylene glycols having a molecular weight of about 6000), certain polypeptides (such as a polyamino acids) and/ or polymers (such as poly-d-lactic acid).
3. Active material(s) and/or excipient(s) which are non-crystalline (amorphous) and which are maintained in the amorphous state throughout subsequent drying, dispensing and final finishing, storage and distribution. Examples include certain saccharides (such as amorphous lactose), certain polyethylene glycols (such as polyethylene glycols having a molecular weight up to 1000), a polyglycan, a polysaccharide (such as a dextran), a cyclodextrin, povidone, micro-fine cellulose, certain polymers (such as potato starch) and a protein.

Compounds in groups 2 and 3 are defined as amorphous for the purposes of the present invention.

The active component or components suitable for use in this invention include , for example:
- A pharmacologically active organic and/or inorganic compound;
- A low molecular weight organic compound such as an amino acid, a vitamin, etc;
- A larger organic molecule, such as a peptide, lipid, polysaccharide, etc;
- A more complex organic molecule, where secondary, tertiary or quaternary structure provides a high molecular weight structure which may comprise several individual polymer chains, such as a polypeptide, polyglycan, polysaccharide, protein including a hormone, enzyme, healing promoter, sub-unit vaccine, etc.; and/or
- An organic and/or inorganic complex which displays high molecular weight, complex structure and large physical size. This group includes a live, attenuated or inactivated prokaryotic and/or eukaryotic cell or virus which may be used, for example, prophylactically or as a vaccine by promoting a systemic immunological response.

Several active components or materials may be incorporated into a single formulation to provide a more effective product. The active component(s) may be augmented by adding a simple or complex compound which may not be active itself but which may potentiate the effects of the active component(s) or act as an adjuvant. The formulation may also include an active component stabiliser.

The active material is preferably apomorphine and/or dihydroergotamine and/or a salt thereof. A suitable salt is apomorphine hydrochloride and/or one or more other water soluble pharmaceutically acceptable salts. In the case of dihydroergotamine, a suitable salt is dihydroergotamine mesylate and/or tartrate and/or one or more other water soluble pharmaceutically acceptable salts.

An excipient to be used in a formulation of this invention must satisfy the crystalline/amorphous parameter given above, and preferably should satisfy one or more of the following parameters:
- Be pharmacologically inert;
- Be compatible with processing requirements;
- Be well tolerated by nasal tissue and nasal physiological function;
- Be non-damaging to the active material;
- Provide a soluble, absorbable product;
- Provide a shelf-stable product; and
- Provide a commercially acceptable product.

Excipient(s) that may be used, subject to the required crystalline/amorphous balance, include a saccharide, a polysaccharide and/or a sugar alcohol.

The term "cyclodextrin" refers to a cyclic oligosaccharide, such as alpha-, beta- and gamma-cyclodextrin and/or a derivative therof, such as methylated beta-cyclodextrin.

The term "saccharide" includes a monosaccharide (such as glucose), a disaccharide (such as lactose, maltose, trehalose, sucrose, and/or saccharose) and a polysaccharide (such as a dextran).

The term "sugar alcohol" refers to a saccharide polyol such as mannitol, sorbitol, inositol and/or xylitol.

The nasal powder formulation according to the invention have the advantage that no preservatives (i.e. bactericides or fungicides) are necessary. Preservatives are known to decrease the ciliary movement, which may be harmful in chronic nasal medication (Hermens W. A. J. J. and Merkus F. W. H. M., Pharm. Res. 1987; 4: 445-449).

The formulation according to the invention can be administered using a nasal insufflator or a passive device. For example, the formulation is placed in a capsule which is set in an inhalation or insufflation device. A needle is penetrated through the capsule to make pores at the top and the bottom of the capsule and air is drawn in by inhalation or blown through the device to force out the powder particles into the patient's nose. The formulation can also be administered in a jet-spray of an inert gas or suspended in liquid organic fluids. The required amount for a nasal administration of a nasal formulation according to the invention may be, for example, between 1 and 50 mg, typically 1 to 20 mg, for example administered as about 5 to 20 mg per nostril.

According to the invention there is also provided a formulation according to the invention for use in therapeutic treatment of a human or animal body by nasal administration. The formulation is preferably for use in treatment of migraine and/or Parkinson's disease and/or sexual dysfunction by nasal administration.

According to the invention there is further provided use of a formulation according to the invention in the manufacture of a medicament for use in therapeutic treatment of a human or animal body by nasal administration. Preferably the medicament is for use in treatment of migraine and/or Parkinson's disease and/or sexual dysfunction by nasal administration.

According to the invention there is also provided a method of medical treatment which method comprises supplying to a human or animal (preferably mammal) patient a therapeutically effective amount of a formulation according to the invention. In the method of the invention, the formulation is preferably supplied to the patient by nasal administration. The method is preferably for the treatment of migraine and/or Parkinson's disease and/or sexual dysfunction in a human patient.

The formulation of the present invention is generally prepared by freeze-drying. The active component(s) and excipient(s) should be compatible with the drying process and should provide a bulk within the processing container to prevent the migration of drying product during processing (ablation). It has been established that by working within the crystalline/amorphous parameter defined above, it is possible to predictably obtain a resultant dried powder which exhibits a particle size suitable for comfortable retention and a fast dissolution of the active material in the nasal mucosa, followed by absorption into the systemic circulation. The dried formulation according to the invention comprises particles which remain stable and uniform throughout processing, final finishing, storage and distribution. The formulation is shelf-stable and free-flowing, presents no problems when dispensed into its final container and is simple to administer by the patient.

The ratio and persistence of the amorphous and crystalline contents of the formulation according to the invention may be determined for compliance with crystalline/amorphous parameter defined above by a thermal analysis technique including differential scanning calorimetry. The particle size distribution pattern of the formulation may be defined by particle size characterisation using a laser diffraction technique with, for example, Mastersizer instrumentation from Malvern Instruments. This laser diffraction powder characterization technique may be carried out directly on a dry powder sample of the formulation (dry analysis) or on a sample of the formulation suspended in a solvent in which the formulation is not soluble (wet analysis). It is necessary to ensure that each sample analysed is fully de-aggregated at the time of characterization and this is best achieved using the wet analysis method. With this method de-aggregation of particle agglomerates can be achieved by the use of dispersing agents, surfactants and/or sonication of the sample prior to analysis and maintained by stirring or recirculation of the sample during analysis. In addition, de-aggregation of the sample can be verified visually under a microscope.

By complying with the crystalline/amorphous parameter defined above and provided that an aqueous formulation of the components is compatible with freeze drying, then the formulation according to the invention preferably has one or more of the following properties:
- A particle size suitable for nasal delivery that can be induced and maintained by freeze-drying;
- The small particle size distribution (finings) wherein small particles generally have a size less than 5µm, is minimized;
- When removed from the freeze-dryer and exposed to atmosphere, the particles of the formulation do not alter in size nor absorb moisture to the extent that the particles aggregate or become sticky, thereby preventing final finishing or dispensing and also influencing pharmacological activity;
- The resultant nasal powder exhibits high solubility, improved nasal absorption and, as a consequence, very high pharmacological activity,

More particularly, the particle size, as measured by laser diffraction, under which 10% by volume of the particles are distributed (D(v,0.1)) is at least 5µm, preferably at least 6µm, more preferably at least 9µm, most preferably at least 10µm, particularly preferably at least 15µm.

The particle size, as measured by laser diffraction, under which 90% by volume of the particles is distributed (D(v,0.9)) is preferably at most 150µm, more preferably at most 125µm, most preferably at most 100µm, particularly preferably at most 80µm, more particularly preferably 50µm, especially 45µm.

The particle size distribution (calculated as the difference between D(v,0.9), and D(v,0.1)) is preferably at most 140µm, more preferably at most 110µm, most preferably 50µm, particularly preferably 40µm.

According to the invention there is also provided a method of preparing a powdered pharmaceutical formulation for nasal delivery which comprises forming a mixed solution of active material and excipient(s) containing: 0.5 - 50 % by wt of active material 50 - 99.5 % by wt of excipient(s),
and freeze-drying the solution so that at least 0.1 % by wt of the freeze-dried blend is in an amorphous state.

In the method of the invention, freezing conditions should preferably be selected to provide:
- An optimal ice crystal structure conducive to maximal sublimation rate;
- The maintenance of a crystalline phase within the matrix; and/or
- The induction of and/or maintenance of an amorphous phase within the matrix.

Selection of suitable freezing conditions will be influenced by
- The chemical nature and concentration of the active component(s) and crystallising or amorphous excipient(s) within the solution or suspension;
- Freeze dryer design and specification;
- Primary container used to process the product; and/or
- Sample fill depth.

Differential scanning calorimetry, differential thermal analysis and resistance analysis may be used to define optimum freezing conditions. From such analysis, we have found it desirable that the product should be frozen at a slow rate or a heat annealing cycle applied to induce or maintain the correct matrix composition. For example a freezing rate of about 0.1 to 0.5°C per minute and a heat annealing cycle comprising, for example: cool product to -45°C at 0.1 - 1.0°C per minute; hold 2 hours, warm to -15°C, hold 2 hours, re-cool to -45°C, hold 2 hours before drying; have been used. These values may be used for guidance, but will vary depending on the formulation of the active material and limitations introduced by the apparatus and other component(s) used in freeze-drying.

For example a suitable drying cycle includes heating directly to 5°C for main drying, increased chamber pressure to 150 mTorr to facilitate heat input and increased final drying temperature to 20°C. Variations on this cycle, designed for specific product/process optimisation include a cycle where shelf temperature was raised to 15°C for the initial phase of main (primary) drying and then progressively reduced to 5°C for the remainder of main (primary) drying with chamber pressure increased up to 300 mTorr to facilitate heat input into product followed by increased shelf temperature to 25°C for final (secondary) drying.

Factors which determine the freeze-drying characteristics of a sample include:
- Then glass transition temperature (Tg') which determines the temperature at which the viscosity of the cooled mass decreases sufficiently so that the sample collapses during freeze-drying.
   Glass transition temperatures have been determined by differential scanning calorimetry or differential thermal analysis;
- Operationally the temperature at which sample collapses during freeze-drying is defined as the collapse temperature (Tc). Collapse temperatures are determined by freeze-drying microscopy. In the absence of complicating factors such as the development of surface skins on the drying sample, collapse and glass transition temperatures are typically similar;
- Skin formation and associated defects, are also determined by freeze-drying microscopy.

The invention is illustrated by way of example with reference to the Figures of the accompanying drawings of which:
**Figure 1** shows a graph showing the dissolution rate of two different formulations of apomorphine nasal powder;
**Figure 2** shows a graph plotting the apomorphine plasma concentration against time for three different comparative doses of apomorphine nasal powder, not produced according to the invention; and
**Figure 3** shows a graph plotting the apomorphine plasma concentration against time for four different doses of apomorphine nasal powder prepared according to the invention.

The following Examples which illustrate the invention are not intended to limit the scope of the claims.

### METHOD EXAMPLE 1

The following freeze-drying cycle has been used effectively for formulations having Tg' or Tc at c. -16 to -18°C. This means that the product temperature should be maintained at c. -23°C (i.e. -18°C plus 5°C for operational safety = -23°C).
Freeze to -45°C
Cooling rate 0.25°C per minute
Hold 120 minutes
Main Drying:
   Shelf Temperature (step 1) -20°C Warming rate 1.0°C per minute Hold 1200 minutes
   Chamber pressure 50 mTorr
Shelf Temperature (step 2) 0°C
Warming rate 1.0°C per minute
Hold 720 minutes
Chamber pressure 50 mTorr
Shelf Temperature (step 3) 5°C
Warming rate 1.0°C per minute
Hold 1000 minutes
Chamber pressure 50 mTorr
Final Drying
Shelf Temperature 15°C
Warming rate 1.0°C per minute
Hold 700 minutes
Chamber pressure 50 mTorr

### FORMULATION EXAMPLES 2-11

The following examples of formulations in accordance with this invention wherein the percentages given are percentages by weight were prepared using the freeze drying cycle of Method Example 1. The allocation of "crystalline" or "amorphous" character is made in the context of the procedures described above and refers to the obtained product and not the starting material.

| | | |
|---|---|---|
| **Example 2.** | Apomorphine HCl | 25% (amorphous state) |
| | Mannitol | 75% (crystalline state) |
| | | |
| **Example 3.** | Apomorphine HCl | 2.5% (amorphous state) |
| | Trehalose | 1% (amorphous state) |
| | Mannitol | 96.5% (crystalline state) |
| | | |
| **Example 4.** | Apomorphine HCl | 10% (amorphous state) |
| | Sucrose | 1% (amorphous state) |
| | Inositol | 89% (crystalline state) |
| | | |
| **Example 5.** | Apomorphine HCl | 50% (amorphous state) |
| | Mannitol | 50% (crystalline state) |

### Examples 2A, 3A, 4A, 5A

- as above but with addition of 1% ascorbic acid to stabilize the apomorphine HCl.

| | | |
|---|---|---|
| **Example 6.** | Dihydroergotamine mesylate | 10% (crystalline state) |
| | Trehalose | 1% (amorphous state) |
| | Mannitol | 89% (crystalline state) |

| | | |
|---|---|---|
| **Example 7.** | Dihydroergotamine mesylate | 10% (crystalline state) |
| | Trehalose | 0.1% (amorphous state) |
| | Mannitol | 89.9% (crystalline state) |

| | | |
|---|---|---|
| **Example 8.** | Dihydroergotamine tartrate | 10% (crystalline state) |
| | Sucrose | 1% (amorphous state) |
| | Inositol | 89% (crystalline state) |

| | | |
|---|---|---|
| **Example 9.** | Dihydroergotamine tartrate | 10% (crystalline state) |
| | Sucrose | 1% (amorphous state) |
| | Sorbitol | 89% (crystalline state) |

| | | |
|---|---|---|
| **Example 10.** | Morphine HCl | 50% (amorphous state) |
| | Sorbitol | 50% (crystalline state) |

| | | |
|---|---|---|
| **Example 11.** | Salmon Calcitonin | 100-200 I.U. (amorphous state) |
| | Trehalose | 0.05 - 0.2mg (amorphous state) |
| | Mannitol to | 5-20mg (crystalline state) |

All the above formulations contain an amount of the active ingredient appropriate for a therapeutic dose on administration of 5-20 mg of powder per nostril.

### EXAMPLES 12-19

The samples detailed in Table 1 below were prepared using the freeze drying cycle described above in Method Example 1.

The particle size distribution of each sample produced by freeze drying was then measured by using a Malvern Instruments Mastersizer laser diffraction machine having a 300mm range lens, a beam length of 14.30 mm and sampler MS7. Hexane was used as the solvent, the wetter was Span 85 and each sample was sonicated for 1 minute before being measured.

The results of the particle size distribution measurements are given in terms of the particle size at which 10%, 50% and 90% by volume of the particles exist which are referred to as D(v,0.1), D(v,0.5) and D(v,0.9). The size difference between D(v,0.9) and D(v,0.1) has also been calculated as this indicates the particle size range. In other words, the lower the size difference, the narrower the particle size distribution curve and the less polydisperse the distribution.

**TABLE 1**

| **Example No.** | **Formulation** | **D(v, 0.1)/µm** | **D(v,0.5)/µ m** | **D(v,0.9)/µ m** | **D(v,0.9) - D(v,0.1) µm** |
|---|---|---|---|---|---|
| 12 | Apomorphine 2.5% w/w (Mannitol + 1% Ascorbic Acid) | 6.52 | 22.40 | 55.12 | 48.60 |
| 13 | Apomorphine 10% w/w (Mannitol + 1% Ascorbic Acid) | 15.42 | 55.88 | 124.24 | 108.82 |
| 14 | Apomorphine 10% w/w (Mannitol + 1% Ascorbic Acid + 0.1% Trehalose) | 5.72 | 19.05 | 40.31 | 34.59 |
| 15 | Apomorphine 10% w/w (Mannitol + 1% Ascorbic Acid + 1% Trehalose) | 6.01 | 19.76 | 40.52 | 34.51 |
| 16 | Apomorphine 25% w/w (Mannitol + 1% Ascorbic Acid) | 10.13 | 28.62 | 54.35 | 44.22 |
| 17 | Apomorphine 50% w/w (Mannitol + 1% Ascorbic Acid) | 5.79 | 20.61 | 41.85 | 36.06 |
| Com. 1 | Dihydroergotamine | 3.42 | 22.27 | 47.62 | 44.20 |
| Com. 2 | Dihydroergotamine | 3.94 | 24.84 | 51.95 | 48.01 |
| Com. 3 | Dihydroergotamine | 3.86 | 31.39 | 77.40 | 73.54 |
| 18 | Dihydroergotamine 5% w/w (Mannitol + 0.1% Trehalose) | 9.21 | 23.09 | 42.36 | 33.15 |
| 19 | Dihydroergotamine 5% w/w (Mannitol + 1% Trehalose) | 9.43 | 23.82 | 44.45 | 35.02 |

| | | | | | |
|---|---|---|---|---|---|
| In this table apomorphine means the HCl salt and dihydroergotamine means the mesylate salt. | | | | | |

Examples 12 to 19 in Table 1 meet the preferred definitions of the D(v, 0.1), D(v, 0.9) and the difference between these two values indicating particle size distributions suited for nasal delivery. Comparative Examples 1 to 3 show that without an amorphous component the formulations fall outside of the preferred definition for D(v, 0.1) demonstrating a high amount of finings. Therefore it is clear that the formulations according to the invention are suitable for nasal administration without any further processing being required This is because they have a narrow particle size range of a size which is ideal for retention in the nasal cavity.

### DISSOLUTION DATA

### COMPARATIVE EXAMPLE 4 & EXAMPLE 20

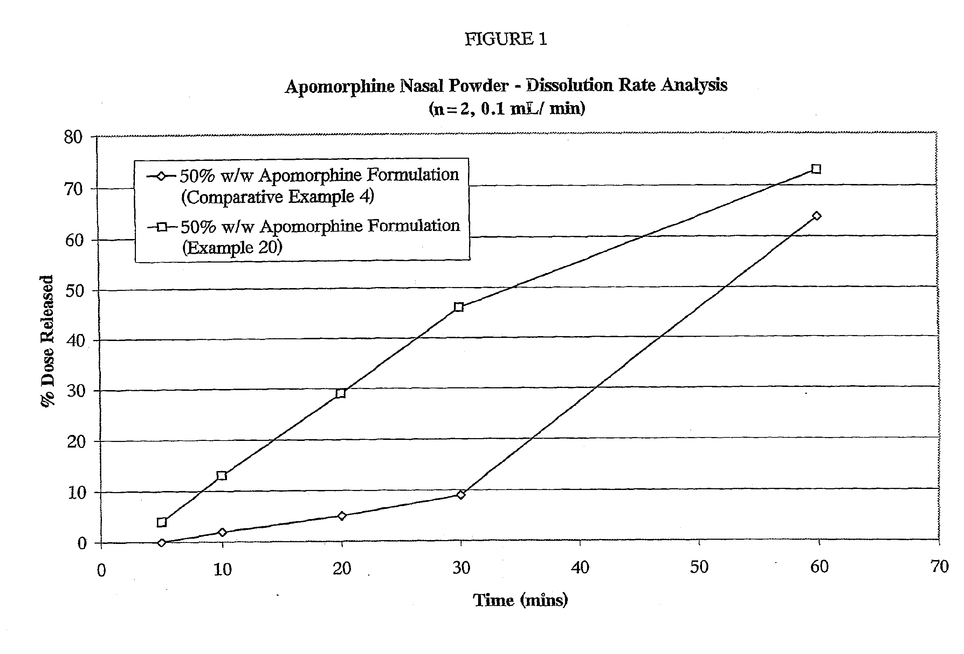

Figure 1 describes the dissolution rate of two different formulations of apomorphine nasal powder (comparative example 4 & example 20). Comparative Example 4 is not according to this invention and was prepared by the mixing of the components of the formulation without freeze drying. The formulation comprised the following components:

### COMPARATIVE EXAMPLE 4

Micronized apomorphine HCl 50%w/w
Ascorbic acid 1% w/w
Dextrose 49% w/w

Example 20 is a formulation in accordance with this invention prepared according to method example 1 and comprised the following components:

### EXAMPLE 20

Apomorphine HCl 50% w/w
Ascorbic acid 1% w/w
Mannitol 49% w/w

Dissolution rate was measured using a micro-dissolution model utilising a flow through approach whereby the dissolution media (sterile water) is introduced at a rate of 0.1 mL/ minute to a single 20mg dose of apomorphine powder held in a glass flow cell. Dissolution is expressed as the percentage of the apomorphine dose in solution against time in minutes.

This model was chosen as over a period of 10-20 minutes the powder is exposed to 1-2 mL of dissolution media. This correlates well with the residence time of particles deposited in the nasal cavity prior to removal by mucociliary clearance and the order of magnitude of volume of fluid typically present in a healthy nose. The dissolution of the sample is measured at specified intervals and the results in Figure 1 are a mean of duplicate determinations.

It is clear that the dissolution rate of formulation prepared in accordance with this invention (example 20) is significantly higher than that seen with the formulation prepared not in accordance with this invention (comparative example 4).

### PHARMACOKINETIC (PK) DATA

### EXAMPLES 21 - 22

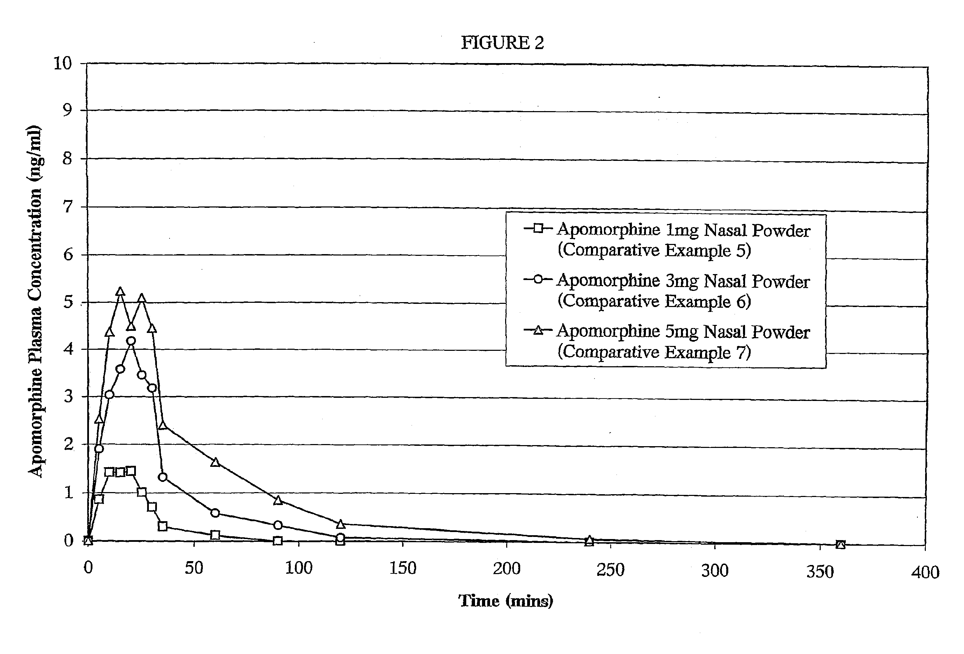

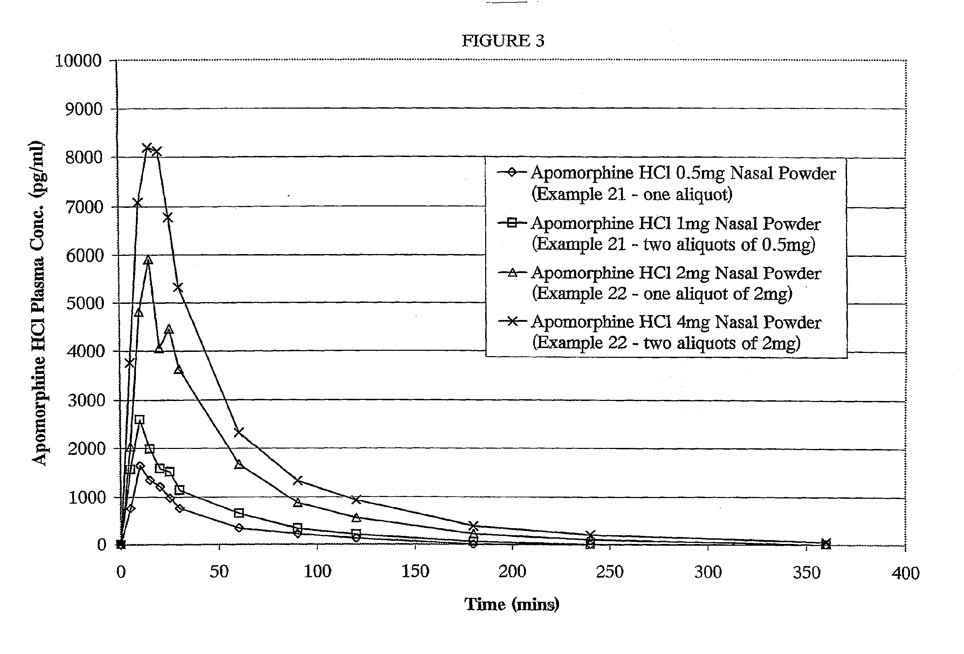

In these examples, the pharmacokinetic profiles of nasal powder formulations not according to the present invention (Figure 2) are compared with those for nasal powder formulations according to the invention (Figure 3).

Three formulations not according to this invention were prepared by simple mixing of the components of the formulation without freeze drying. The three formulations comprised the following components:

### COMPARATIVE EXAMPLE 5

Micronized apomorphine HCl 5% w/w
Ascorbic acid 1% w/w
Dextrose 94% w/w

### COMPARATIVE EXAMPLE 6

Micronized apomorphine HCl 15% w/w
Ascorbic acid 1% w/w
Dextrose 89% w/w

### COMPARATIVE EXAMPLE 7

Micronized apomorphine HCl 25% w/w
Ascorbic acid 1% w/w
Dextrose 74% w/w

These three formulations were administered to 6 healthy male subjects in single 20mg aliquots to one nostril facilitating delivery of apomorphine HCl doses of 1mg, 3mg and 5mg respectively for comparative examples 5-7. Each dose was administered on a separate day in order of ascending dose and blood samples were collected at regular intervals over a 6 hour period following dosing. Blood samples were measured using validated HPLC methodology (Priston M.J. and Sewell G.J., Journal of Chromatography 681: 161-167).

Two further formulations in accordance with this invention were prepared using the freeze drying method previously described in Method example 1 and comprised the following components:

### EXAMPLE 21

Apomorphine HCl 2.5% w/w
Ascorbic acid 1% w/w
Trehalose 1% w/w
Mannitol 95.5% w/w

### EXAMPLE 22

Apomorphine HCl 10% w/w
Ascorbic acid 1% w/w
Mannitol 89% w/w

These two formulations (examples 21 & 22) were administered to 6 healthy, male subjects as single 20 mg aliquots in one nostril (facilitating delivery of apomorphine HCl doses of 0.5mg and 2mg respectively) and as 40 mg aliquots comprising a single 20mg aliquot in each nostril (facilitating delivery of apomorphine HCl doses of 1mg and 4mg respectively). Each dose was administered on a separate day in order of ascending dose and blood samples were collected at regular intervals over a 6 hour period following dosing. Blood samples were measured using validated proprietary LC/MS-MS methodology at Chemical Synthesis Services, 38 Castleroe Road, Coleraine, Northern Ireland.

The PK profiles in Figures 2 and 3 show that the formulations according to the invention are absorbed much more efficiently and deliver much higher blood levels (almost twice as high) than those outside of the invention. This performance is clearly advantageous and shows that the nasal administration route is viable with the formulations according to the invention.

### EXAMPLE 23

Apomorphine is currently available as an aqueous solution for subcutaneous injection or infusion. It is indicated in Parkinson's disease (PD) for the treatment of motor fluctuations refractory to other medications. These motor fluctuations (otherwise referred to as the 'on/ off' phenomenon') are characterised by 'off' periods during which patients experience profound episodes of one or more of bradykinesia/ akinesia, tremor and rigidity.

In this example, it was determined whether a single-dose of intra-nasal apomorphine powder (5mg) was effective in reversing an 'off' period in subjects with PD known to respond to a single dose of ≤ 5mg subcutaneous apomorphine. The nasal powder formulation used in this study was in accordance with this invention prepared using the method of Method example 1 and comprised the following components:
Apomorphine HCl 25% w/w
Ascorbic acid 1% w/w
Mannitol 74% w/w

Six subjects with PD complicated by motor fluctuations were studied, 5 male (mean age 64 years, range 57-70 years), 1 female (age 59 years). The mean duration of disease was 13 years (range 7-24 years). The speed of onset and duration of response was determined on two separate days following, on the first day, their normal dose of subcutaneous apomorphine and, on their second day, intra-nasal apomorphine powder (5mg). On each dosing day the subjects attended the clinic and their normal oral medication was withheld until an 'off' episode developed. Each subject then received the designated apomorphine dose (subcutaneous or nasal). The abbreviated Unified PD Rating Scale (UPDRS) was used to assess motor performance at regular intervals following each apomorphine dosing. The time for the 'off' period to be fully alleviated and the duration of this response were determined primarily by patient feedback and supported by clinical judgement and the UPDRS scores. If a patient failed to respond to either medication within 30 minutes, the patients were rescued with subcutaneous apomorphine and returned to their oral medications.

The results are summarised in Table 2.

**TABLE 2**

| Latency and duration of 'on'state of apomorphine administered by subcutaneous and intra-nasal route. | | | | | | |
|---|---|---|---|---|---|---|
| Subject | Doses of apomorphine (mg) | | Latency for 'on' response (min) | | Duration of 'on' response (min) | |
| | s/c | in | s/c | in | s/c | in |
| 1. | 2 | 5 | 20 | 14 | 96 | 143 |
| 2. | 4 | 5 | 14 | 5 | 65 | 93 |
| 3. | 2 | 5 | 20 | 19 | 75 | 100 |
| 4. | 4 | 5 | 14 | 27 | 48 | 153 |
| 5. | 4 | 5 | 30 | 30 | 43 | 51 |
| 6.* | 5 | 5 | 61 | 11 | 46 | 19 |
| | | **Mean** | **27** | **18** | **62** | **93** |

| | | | | | | |
|---|---|---|---|---|---|---|
| s/c = subcutaneous in = intra-nasal * Subject 6 required an increased subcutaneous apomorphine dose (5mg) 30 minutes after receiving their usual dose (4mg) to achieve 'on' state. The latency of response was calculated from the initial apomorphine dose. The response of subject 6 to intra-nasal dosing was incomplete with the subject stating that they felt 'approximately 80% on'. | | | | | | |

In all subjects, intranasal apomorphine powder (5mg) alleviated the 'off' episode. The mean latency of response after intra-nasal dosing was 18 minutes (range 5-30 minutes) compared to 27 minutes (range 14-61 minutes) after subcutaneous dosing (2-5mg). The mean duration of response after intra-nasal dosing was 93 minutes (range 19-153 minutes) compared to 62 minutes (range 43-96 minutes) after subcutaneous dosing. No serious local or systemic reactions were demonstrated after intra-nasal apomorphine dosing.

Following this study five of these subjects used the intra-nasal apomorphine powder (5mg) twice a day for 7 days to replace two of their daily apomorphine subcutaneous injections. In all cases the nasal powder effectively replaced the injections and no significant adverse events were reported.

The results of this study demonstrate the rapid efficacy of a formulation encompassing the principles described in this invention.

## Claims

1. A powdered pharmaceutical formulation suitable for nasal delivery which is a freeze-dried blend of active material and excipient(s) containing:
0.5-50% wt. of active material;
50-99.5% wt. of excipient(s);
and in which at least 0.1% wt. of the blend is an amorphous state.

2. A formulation according to claim 1, in which from 0.1, preferably from 0.5, more preferably from 1 to 50% wt. of the freeze-dried blend is in an amorphous state.

3. A formulation according to claim 1, wherein said powdered formulation is unmilled, having a particle size measured by laser diffraction under which 10% vol. of the particles are distributed of at least 5µm.

4. A formulation as claimed in claim 1, wherein said excipient(s) are selected from sodium chloride, potassium chloride, amino acids, sugar alcohols, monosaccharides, disaccharides, trisaccharides, polyethyleneglycols, polypeptides, poly-d-lactic acid, polyglycan, povidone, micro-fine cellulose, potato starch and protein.

5. A formulation according to claim 1, containing:
a) 0.5-50% wt. of active material in amorphous state;
50-99.5% wt. of excipient in crystalline state;
0-5% wt. of excipient in amorphous state;
b) 0.5-50% wt. of active material in crystalline state;
50-99.4% wt. of excipient in crystalline state;
0.1-5% wt. of excipient in amorphous state; or
c) 0.5-25% wt. of active material in amorphous or crystalline;
75-99% wt. of excipient, in crystalline state;
0.5-5% wt. of excipient in amorphous state
wherein preferably said excipient in amorphous state is one or more excipients selected from the group consisting of glutamine, serine, a monosaccharide, a disaccharide, a trisaccharide, polyethyleneglycols having a molecular weight up to 1000, polyethyleneglycols having a molecular weight of about 6000, a polyamino acid, poly-d-lactic acid, a polyglycan, povidone, microfine cellulose and potato starch.

6. A formulation according to claim 1, containing:
a) 2.5-50% wt. apomorphine HCl or other water soluble pharmaceutically acceptable salts in amorphous state;
50-97.5% wt. of excipient(s); or
b) 2.5-50% wt. dihydroergotamine mesylate and/or tartrate and/or other water soluble pharmaceutically acceptable salts in crystalline state;
50-97.5% wt. of excipient(s).

7. A formulation according to any of claims 1 to 6 in which:
a) a saccharide is used to provide an excipient in amorphous state, wherein preferably said saccharide is selected from the group consisting of glucose, sucrose, trehalose, lactose, maltose and saccharose; or
b) a sugar alcohol is used to provide an excipient in crystalline state, wherein preferably said sugar alcohol is selected from the group consisting of mannitol, inositol, sorbitol and xylitol.

8. A formulation according to any one of the preceding claims having:
a) a particle size measured by laser diffraction under which 10% by volume of the particles are distributed of at least 5 µm, preferably at least 6 µm, more preferably at least 9 µm, most preferably at least 10 µm, particularly preferably at least 15 µm;
b) a particle size measured by laser diffraction under which 90% by volume of the particles is distributed of at most 150 µm, more preferably at most 125 µm, most preferably at most 100 µm, particularly preferably at most 80 µm, more particularly preferably 50 µm, especially 45 µm; or
c) a particle size distribution of at most 140 µm, more preferably at most 110 µm, most preferably 50 µm, particularly preferably 40 µm.

9. A formulation according to any one of the preceding claims for use in therapeutic treatment of a human or animal body by nasal administration, wherein preferably the formulation is for use in the treatment of migraine and/or Parkinson's disease and/or sexual dysfunction by nasal administration.

10. A method of preparing a powdered pharmaceutical formulation for nasal delivery which comprises forming a mixed solution of active material and excipient(s) containing:
0.5-50% wt. of active material;
50-99.5% wt. of excipient(s);
and freeze-drying the solution so that at least 0.1 % wt. of the freeze-dried blend is in an amorphous state.

11. A method as claimed in claim 10, wherein said powdered formulation is unmilled, being the direct product of said freeze-drying step and having a particle size measured by laser diffraction under which 10% vol. of the particles are distributed of at least 5 µm.

12. A method as claimed in claim 10, wherein said excipient(s) are selected from sodium chloride, potassium chloride, amino acids, sugar alcohols, monosaccharides, disaccharides, trisaccharides, polyethyleneglycols, polypeptides, poly-d-lactic acid, polyglycan, povidone, micro-fine cellulose, potato starch and proteins.

13. A method according to claim 10, in which
a) the active material freeze dries to a crystalline state and the mixed solution contains:
0.5-50% wt. of active material;
50-99.4% wt. of excipient which freeze dries to a crystalline state;
0.1-5% wt. of excipient which freeze dries to an amorphous state or
b) the active material freeze dries to an amorphous state and the mixed solution contains:
0.5-50% wt. of active material;
50-99.5% wt. of excipient which freeze dries to a crystalline state;
0-5% wt. of excipient which freeze dries to an amorphous state.
wherein said excipient in amorphous state is one or more excipients selected from the group consisting of glutamine, serine, a monosaccharide, a disaccharide, a trisaccharide, polyethyleneglycols having a molecular weight up to 1000, polyethyleneglycols having a molecular weight of about 6000, a polyamino acid, poly-d-lactic acid, a polyglycan, povidone, microfine cellulose and potato starch.

14. A method according to claim 10, in which the mixed solution contains:
a) 0.5-25% wt. of active material which freeze dries to an amorphous or crystalline state;
75-99% wt. of excipient which freeze dries to a crystalline state;
0.5-5% wt. of excipient which freeze dries to an amorphous state;
b) 2.5-50% wt. apomorphine HCl or other water soluble, pharmaceutically acceptable salts in amorphous state;
50-97.5% wt. of excipient(s); or
c) 2.5-50% wt. dihydroergotamine mesylate and/or tartrate and/or other water soluble, pharmaceutically acceptable salts in crystalline state;
50-97.5% wt. of excipient(s).

15. A method according to any of claims 10 to 14 in which:
a) a saccharide is used to provide an excipient in amorphous state, wherein preferably said saccharide is selected from the group consisting of glucose, sucrose, trehalose, lactose, maltose and saccharose; or
b) a sugar alcohol is used to provide an excipient in crystalline state, wherein preferably said sugar alcohol is selected from the group consisting of mannitol, inositol, sorbitol and xylitol.
